# EUROPEAN PATENT APPLICATION

(11) **EP 4 350 210 A1**
(43) Date of publication of application: **10.04.2024**
(21) Application number: 22199654.9
(22) Date of filing: 04.10.2022
(51) Int. Cl.: F21V 15/01, F21V 29/70, F21V 17/00, F21V 31/00, F21V 17/16, A61B 90/30, F21W 131/205

(54) **SURGICAL LIGHTING ASSEMBLY**

(71) Applicant: Baxter Medical Systems GmbH + Co. KG, 07318 Saalfeld (DE)
(72) Inventor: Hanuschka, Lars, 07318 Saalfeld (DE); Hänel, Martin, 07318 Saalfeld (DE)
(74) Representative: Dundas, Gavin Turner

(57) **Abstract**

A surgical lighting assembly (100) comprises an upper housing (110), with an upper rim arranged (120) around at least a portion of a perimeter of the upper housing, and a lower housing (130), with a lower rim (140) arranged around at least a portion of a perimeter of the lower housing. The upper housing and the lower housing are configured to fit together around their perimeters to define a chamber (250) therebetween. The lighting assembly further comprises a light source (270) in the chamber, a sealing member (170) located between the perimeters of the upper and lower housings, and a plurality of clamping strips (190). The clamping strips are configured to clamp the upper rim to the lower rim around the perimeter of the housings, so that the upper housing is clamped to the lower housing in a sealed configuration.

## Description

### TECHNICAL FIELD

The present invention relates to a surgical lighting assembly for use in a medical setting, such as in an operating theatre or surgical suite. In particular, the invention relates to a surgical lighting assembly with an improved ingress protection (IP) rating.

### BACKGROUND

Surgical lights are lighting fixtures designed for use during surgical procedures, to provide illumination to a desired area. Due to the particular demands in a medical or surgical setting, surgical lights are subject to a large number of requirements in terms of their construction and lighting performance. For example, surgical and examination lighting devices must meet a number of standards in terms of the brightness, colour rendition, light field diameter, and homogeneity of the light produced.

Another key consideration in surgical lighting relates to the ease with which the light fitting may be cleaned and disinfected. As strong liquid disinfectants are commonly used to disinfect and decontaminate surgical suites, it is desirable that as well as meeting the required lighting standards, surgical lights should also exhibit good ingress protection (IP) characteristics to protect the internal components from damage during cleaning.

One surgical lighthead that is well known in the prior art is the iLED (RTM) 7 Surgical Light sold by Trumpf Medical, a subsidiary of Hill-Rom. Prior art surgical lamps are also disclosed in CN201589205U and CN209341155U.

### STATEMENT OF INVENTION

The invention provides a surgical lighting assembly, as defined in the appended independent claims, to which reference should now be made. Preferred or advantageous features of the invention are set out in dependent subclaims.

According to a first aspect of the present invention, there is provided a surgical lighting assembly, comprising an upper housing, with an upper rim arranged around at least a portion of a perimeter of the upper housing, and a lower housing, with a lower rim arranged around at least a portion of a perimeter of the lower housing. The upper housing and the lower housing are configured to fit together around their perimeters to define a chamber therebetween. The lighting assembly further comprises a light source arranged in the chamber between the housings, a sealing member located between the perimeters of the upper and lower housings, and a plurality of clamping strips. The clamping strips are configured to clamp the upper rim to the lower rim around the perimeter of the housings, so that the upper housing is clamped to the lower housing in a sealed configuration.

When the upper housing and the lower housing fit together around their perimeters, a joint is formed between the housing perimeters. The sealing member is preferably arranged in the joint between the upper and lower housings.

The surgical lighting assembly may alternatively be called a surgical lighthead, a surgical lamp, or an operating light.

In the prior art, surgical lightheads have typically suffered from poor ingress protection (IP) ratings. In order to protect lighthead housings against ingress by dust or liquid, prior art lightheads have typically comprised multiple seals at joints in the housing, and a large number of fastener (typically screws) which could be tightened to fasten components of the housing together. One drawback of this arrangement is the time taken to open and re-seal the lighthead housing when maintenance is required, as each fastener must be manually undone and then re-done. Another drawback is that the variable-tightness of each fastener can make the seals between housing components unreliable, as some fasteners may be tightened more than others. A further drawback is that the use of multiple seals increases the risk that not all seals may be positioned precisely every time the housing is opened and re-sealed, and that extra time and care must be spent to align all of the seals when the housing is sealed.

The surgical lighting assembly of the present invention provides a simplified construction which also results in an improved ingress protection (IP) rating.

Ingress Protection ratings are defined by International Ingress Protection Code - BS EN 60529.

In the lighting assembly of the present invention, the upper housing is clamped to the lower housing in a sealed configuration thanks to a sealing member located between the perimeters of the upper and lower housings, and a plurality of clamping strips. The clamping strips provide a mechanical fixation between the upper and lower housings.

The use of clamping strips rather than screw fasteners provides significant benefits. One benefit of this is that the clamping strips provide a much more uniform clamping pressure than screw fasteners, where the pressure is variable depending on how tightly each individual screw is screwed into place. The clamping strips are also advantageously simple and quick to both apply and remove from the housing perimeters, so that the lighting assembly can be disassembled and reassembled quickly and easily for repairs or part replacement if needed.

The upper housing may advantageously be provided as a one-piece moulded polymer shell. The lower housing may advantageously be provided as a one-piece moulded polymer shell. Due to the manufacturing tolerances involved in moulding polymer housings of the sizes required for surgical lightheads, the perimeters of the upper and lower housings will not necessarily fit flush with one another every time. In this situation the use of screws to fasten the housing perimeters together may be difficult, as the seats for the screws on each housing may not align perfectly each time. By using clamping strips, however, the effects of misalignments between the housings due to manufacturing tolerances are minimised, as there is no need for screw seats to be precisely aligned, and the clamping strips pull the upper and lower rims together along the entire length of the clamping strips.

Particularly advantageously, the surgical lighting assembly may achieve an IP54 rating as a result of the high level of sealing produced by the sealing member and the clamping strips around the perimeter of the upper and lower housings.

An Ingress Protection 54 (IP54) rating signifies that a lighting assembly is dust protected, such that it prevents ingress of dust sufficient to cause damage, and that it is protected against water splashing from any angle.

Preferably the outer perimeters of the upper and lower housings are only fastened together by clamping strips, and not by any other mechanical fasteners. Particularly preferably, the outer perimeters of the upper and lower housings are not fastened to one another by any screws.

Each clamping strip may preferably comprise a profile strip configured to fit over the upper rim and the lower rim. In preferred embodiments, the clamping strip may be a profile strip, for example a metal profile such as an aluminium profile. The clamping strip may comprise a channel shaped to receive the upper rim and the lower rim, and to hold the upper rim and the lower rim together inside the channel.

In a particularly preferred embodiment, the clamping strip comprises a T-shaped channel, the T-shaped channel being configured to receive the upper rim and the lower rim therein. The T-shaped channel is oriented with the arm of the T directed inwards, and the stem of the T forming the opening into the channel, so that when the clamping strip is in position, the upper and lower rims are received in the arm of the T, with the upper and lower rims pointing in opposite directions along the arm of the T-shaped channel.

The upper rim preferably projects upwards, in a direction away from the joint between the perimeters of the upper and lower housings when they are fitted together. The lower rim preferably projects in the opposite direction from the upper rim. The lower rim preferably projects downwards, in a direction away from the joint between the perimeters of the upper and lower housings when they are fitted together.

The terms "upper" and "lower" are used herein to denote the orientation of the surgical lighthead during normal use. Surgical lighting assemblies are typically suspended above the area requiring illumination, with light projecting downwards from the light source onto the area requiring illumination. Thus in this context the lower housing is the side of the housing facing downwards towards the area being illuminated by the lighthead, while the upper housing is the side of the housing facing upwards away from the area being illuminated.

The surgical lighting assembly comprises an upper rim arranged around at least a portion of a perimeter of the upper housing, and a lower rim arranged at least a portion of the perimeter of the lower housing. The positions and number of upper and lower rims may be varied, while still providing the benefits of the present invention.

In some embodiments, for example, the upper housing may comprise a single continuous upper rim which extends around the perimeter of the upper housing. Likewise the lower housing may comprise a single continuous lower rim which extends around the perimeter of the lower housing. The upper housing may comprise a plurality of upper rims, each upper rim being arranged to extend around a portion of the perimeter of the upper housing, and/or a plurality of lower rims, each lower rim being arranged to extend around a portion of the perimeter of the lower housing.

Where the upper and lower rims are continuous, the clamping strips may be resilient, and clampable over the upper and lower rims by flexing the strips to pass over the upper and lower rims before the strips flex back into their normal shape and clamp the rims together.

In particularly preferred embodiments, the upper and lower rims do not extend continuously around the entire perimeter of their respective housing. In preferred embodiments, the upper housing may comprise a plurality of upper rims, each extending around a portion of the perimeter of the upper housing. Likewise, the lower housing may comprise a plurality of lower rims, each extending around a portion of the perimeter of the lower housing. The plurality of upper rims may be arranged with gaps or spaces between adjacent upper rims. Likewise, the plurality of lower rims may be arranged with gaps or spaces between adjacent lower rims. The presence of gaps or spaces between adjacent rims may advantageously provide space for the clamping strips to be slid over a pair of upper and lower rims from one end. As the clamping strips need not be resilient in order to snap over the rims, this advantageously allows the clamping strips to be made thicker and/or more rigid, which may in turn produce a stronger and more reliable clamping force between the housings.

Preferably the upper rim or rims match the lower rim or rims. In other words, the number and positions of the upper rims preferably correspond to the number and positions of the lower rims, so that when the upper and lower housings are fitted together around their perimeters, each upper rim is aligned with a corresponding lower rim.

The plurality of upper rims may extend around at least 75%, or at least 80%, or at least 85%, or at least 90% of the perimeter of the upper housing. The plurality of lower rims may extend around at least 75%, or at least 80%, or at least 85%, or at least 90% of the perimeter of the lower housing. This may advantageously allow the upper and lower housings to be clamped together by the clamping strips over at least 75%, or at least 80%, or at least 85%, or at least 90% of the perimeter of the housings, which produces extremely an effective clamping force and high sealing performance.

The size and length of the clamping strips may be varied while still providing the high clamping force and good ingress protection characterising the present invention. In some preferred embodiments, however, the clamping strips may have a length of at least 10 cm, or at least 12 cm, or at least 15 cm. When a clamping strip is in position and clamping together an upper rim and a lower rim, the housing perimeters are clamped over the entire length of the clamping strip. Compared to the single connection point provided by a fastener such as a screw, the relatively long clamping strips thus provide clamping pressure onto the housing perimeters over at least 10 cm, or 12 cm, or 15 cm per clamping strip. This advantageously creates a very secure clamping effect, and a high level of ingress protection.

The housings of the lighting assembly may take a variety of shapes within the scope of the present disclosure. The majority of prior art surgical lamps have been circular in shape, while the iLED (RTM) 7 is hexagonal in shape. The perimeters of the upper and lower housings are preferably matching, so that the upper and lower housings may be fastened together around their perimeters.

In particularly preferred embodiments, however, the upper and lower housings are in the shape of regular polygons, such that the perimeters of the housings are polygonal in shape. Regular polygons include triangles, squares, pentagons, hexagons, heptagons, octagons, nonagons and decagons.

The lighting assembly preferably comprises at least one clamping strip for each edge of the polygonal housings. Thus if the housing perimeters take the shape of a triangle, the assembly preferably comprises at least three clamping strips. If the housing perimeters take the shape of a square, the assembly preferably comprises at least four clamping strips. A pentagonal assembly preferably comprises at least five clamping strips, a hexagonal assembly preferably comprises at least 6 clamping strips, and so on.

Particularly preferably the lighting assembly comprises two or more clamping strips for each (straight or relatively straight) edge of the polygonal housings. For example the perimeters of the upper and lower housings may be hexagonal, and the assembly may include 12 clamping strips, so that each of the six edges of the hexagonal housings are clamped by two clamping strips. Likewise if the perimeters of the upper and lower housings are octagonal, the assembly may include 16 clamping strips, so that each of the eight edges of the octagonal housings are clamped by two clamping strips. Providing at least two clamping strips per edge of the housing may advantageously provide a very secure clamping force along each edge, and thus around the whole perimeter of the housings.

The plurality of clamping strips are preferably arranged symmetrically and/or regularly around the perimeter of the housings. The rims may also be arranged symmetrically around the perimeters of the housings, to ensure that the clamping strips are positioned symmetrically when clamped over the rims. A symmetrical arrangement of clamping strips advantageously produces an even clamping force around the whole perimeter of the housings, to ensure that the assembly is reliably sealed.

The surgical lighting assembly may comprise a bumper which is configured to extend around the perimeters of the upper and lower housings. The bumper is preferably configured to cover the joint between the upper and lower housings, and to cover the plurality of clamping strips. Particularly preferably the bumper is formed from a resilient material, for example a resilient polymer material. The bumper advantageously provides shock absorption which protects the assembly from damage during use. By covering the joint between the housings and the mechanical clamping strips, the bumper advantageously makes the assembly easier to clean, by preventing liquid and debris from becoming caught in and around the clamping strips and the sealed joint between housings. Although the sealing and ingress protection rating is already achieved by the sealing member and the clamping strips, the bumper may provide an additional layer of ingress protection, by preventing dust and liquid from coming into contact with the joint and the clamping strips.

The bumper may be held in position around the perimeters of the housings by one or more rims on at least one of the housings.

In a preferred embodiment, the assembly comprises a second upper rim arranged around at least a portion of the perimeter of the upper housing, and a second lower rim arranged around at least a portion of the perimeter of the lower housing. The bumper may comprise a channel, or a pair of channels, configured to receive the second upper rim and the second lower rim. In a preferred embodiment, the bumper comprises a T-shaped channel, in which the arm of the T is configured to receive the second rims. The second rims may function to locate the bumper in position over the joint and clamping members, and to hold it in place unless enough force is applied to pull the bumper off over the second rims.

The bumper preferably comprises a clamping member channel for receiving the clamping members when the bumper extends around the outer edge of the housings. The clamping member channel may be connected to the channel for receiving the second upper and lower rims.

Like the iLED (RTM) 7, the surgical lighting assembly of the present invention may comprise an opening in the upper and lower housings, which forms a tunnel which extends through the lighthead from its top surface to its bottom surface. This opening may advantageously make the light ideal for conventional or laminar airflow systems. Where the opening is present, the upper and lower housings are sealed around the opening, such that the chamber extends as a ring bounded by the opening on the inside, and the perimeter of the housings on the outside.

The upper housing may be sealed to the lower housing around the opening. Alternatively, the assembly may comprise a separate hollow cylindrical member configured to connect to the opening in the upper housing at an upper end, and to connect to the opening in the lower housing at a lower end. The cylindrical member may be sealingly connected to the upper and lower housings by a plurality of sealing rings and a plurality of mechanical fasteners, for example screws. When the cylindrical member is connected to the openings in the upper and lower housings, the hollow centre of the cylindrical member forms the tunnel-shaped opening through the housings.

The surgical lighting assembly may comprise a heat sink arranged in the chamber on the underside of the upper housing. The heat sink is preferably metal, and may be formed of a ring of metal attached to the underside of the upper housing. The heat sink preferably extends in a ring around the opening through the lighthead, so that it is contained in the chamber when the lighthead is assembled with the housings clamped together. The heat sink may comprise a plurality of cooling fins configured to distribute heat over the upper housing. This form of heat sink is highly effective at absorbing heat from electronic components inside the chamber formed between the housings, and dissipating the absorbed heat over the upper housing to increase the efficiency of the heat loss to air outside the lighthead. The arrangement of providing the heat sink inside the lighthead housings is advantageously compact and easy to clean.

The heat sink may preferably be configured to contact the underside of the upper housing over at least 20%, or at least 25%, or at least 30% of the surface area of the underside of the upper housing.

As well as the light source which is located inside the chamber defined by the upper and lower housings, a variety of sensors and control electronics for controlling the light source are also provided inside the chamber.

In preferred embodiments of the present invention, an outer surface of the upper housing or the lower housing comprises a data connection port connected to control electronics housed in the chamber. This data connection port advantageously acts as an external service interface for the electronic components inside the chamber, so that data may be exchanged, and for example software may be updated, without the need to disassemble the lighthead to access the electronic components inside. This greatly reduces the chances of dust and liquid ingress into the chamber during disassembly and reassembly, and reduces downtime for the lighting assembly.

Similarly to the iLED (RTM) 7, the lighting assembly of the present invention preferably comprises a sterile handle configured to project underneath the lower housing, so that a person involved in a surgical operation, for example the surgeon, can manipulate the lighting assembly using the sterile handle.

The surgical lighting assembly preferably comprises a plurality of handles or grips on the upper housing. Particularly preferably, the plurality of handles are ridges moulded integrally into the housing. These moulded handles or grips may advantageously provide non-sterile handling points so that during use of the lighting assembly in a surgical setting, attendants whose hands have not been sterilised can move the position of the lighting assembly without having to touch the sterile handle. This advantageously helps to avoid contamination caused by both sterile and non-sterile hands touching the same parts of the lighting assembly to manoeuvre the lighting assembly into the desired position. By moulding these non-sterile handles as ridges integral with the upper housing, it is ensured that these handling points are well separated from the sterile handle beneath the lower housing, and that the handles are easy to clean and do not comprise any crevices to harbour bacteria.

The upper housing preferably comprises or consists of a one-piece moulded polymer shell.

In a preferred embodiment, the lower housing comprises or consists of a one-piece moulded polymer shell. The moulded polymer shell comprises a transparent window for transmitting light from the light source. Preferably the polymer shell is a polycarbonate shell. By providing the lower housing as a one-piece moulded shell, the need for seals between the transparent window and a lower housing surrounding the window is eliminated. The one-piece moulded lower housing is easier to clean, simpler to assemble and disassemble with the upper housing, and provides a higher IP rating as there are fewer joints and seals for dust and liquid to enter the chamber inside the housing.

The lower housing may comprise a one-piece moulded shell of transparent polymer, with a separately moulded opaque frame configured to fit over the transparent shell so that the transparent window is defined in an aperture in the opaque frame.

Preferably the lower housing comprises a polymer shell, particularly preferably a 2k moulded polymer shell. Thus the lower housing may consist of a one-piece moulded polymer shell. 2K injection moulding allows the production of two or three different plastic materials in one solid part. This advantageously allows the transparent window to be moulded in one piece with an opaque portion of the lower housing, for example an opaque frame surrounding the transparent window.

The lower housing preferably comprises a sensor housing moulded integrally into the housing. The sensor housing may contain sensors configured to sense one or more parameters relating to the lighting provided by the light source.

The light source may comprise a plurality of LEDs. The light source is preferably configured to provide an adjustable illumination level, an adjustable pattern size, and an adjustable colour temperature of the emitted light.

The housing preferably comprises a frame connector configured to connect to a supporting frame. The supporting frame may also be called a yoke or an arm, and is preferably a multi-jointed frame assembly which is adjustable to support the lighting assembly in a variety of positions, and at a variety of angles.

Preferably the lower housing comprises the frame connector. The upper housing is preferably removable from the lower housing while the lower housing is connected to the supporting frame. The upper housing may be removed from the lower housing by removing the bumper (if present), removing the clamping strips, and removing any fastenings around the opening through the housings. As the frame connector is connected to the lower housing, the upper housing may advantageously be removed without having to disconnect the entire assembly from the supporting frame. This may advantageously speed up repairs and reduce downtime, as well as reducing the risk of damage while mounting or detaching the assembly onto the support frame.

The frame connector may comprise a rotatable connection for receiving power and hard-wired data transfer from the frame. A hard-wired data connection is preferably provided as a more reliable alternative to the WLAN communications system employed in some prior art lightheads. The rotatable connection may be provided by, for example, a 9-pin connection which is rotatable around 360 degrees.

The frame connector may comprise a seal configured to prevent dust or liquid ingress at the frame connector.

The sealing member preferably comprises a single strip of resilient material extending around the joint between the perimeters of the upper and lower housings. By providing the sealing member as a single strip, the step of sealing the housings together when assembling the device is greatly simplified, as only the one seal must be correctly positioned in order to seal the assembly around the entire perimeter of the housings. This advantageously increases the reliability of the sealing, as there are fewer sealed joints at which dust or water ingress could occur.

Particularly preferably at least one of the upper or lower housing comprises a peripheral channel for receiving the sealing member. The peripheral channel preferably extends around the perimeter of the housing, and is configured so that when the sealing member is received in the channel and the upper and lower housings are fitted together, the sealing member is held in the joint between both housings, and extends around the entire perimeter to provide a continuous seal between the housings.

### Second Aspect

In a second aspect there is provided a surgical lighting assembly, comprising:
an upper housing and a lower housing, the upper housing and the lower housing being configured to fit together to define a chamber therebetween;
the lighting assembly further comprising:
   a light source in the chamber;
   in which the upper housing comprises a one-piece moulded polymer shell, and in which the lower housing comprises a one-piece moulded polymer shell comprising a transparent window for transmitting light from the light source.

Preferably the lower housing comprises a polycarbonate shell, preferably clear polycarbonate. By providing the lower housing as a one-piece moulded shell, the need for seals between the transparent window and a lower housing surrounding the window is eliminated. The one-piece moulded lower housing is easier to clean, simpler to assemble and disassemble with the upper housing, and provides a higher IP rating as there are fewer joints and seals for dust and liquid to enter the chamber inside the housing.

The lower housing may comprise a one-piece moulded shell of transparent polymer, with a separately moulded opaque frame configured to fit over the transparent shell so that the transparent window is defined in an aperture in the opaque frame.

Particularly preferably the lower housing comprises a 2k moulded polymer shell. Thus the lower housing may consist of a one-piece moulded polymer shell. 2K injection moulding allows the production of two or three different plastic materials in one solid part. This advantageously allows the transparent window to be moulded in one piece with an opaque portion of the lower housing, for example an opaque frame surrounding the transparent window.

Like the iLED (RTM) 7, the surgical lighting assembly of the present invention may comprise an opening in the upper and lower housings, which forms a tunnel which extends through the lighthead from its top surface to its bottom surface. This opening may advantageously make the light ideal for conventional or laminar airflow systems. Where the opening is present, the upper and lower housings are sealed around the opening, such that the chamber extends as a ring bounded by the opening on the inside, and the perimeter of the housings on the outside.

The upper housing may be sealed to the lower housing around the opening.

Alternatively, the assembly may comprise a separate hollow cylindrical member configured to connect to the opening in the upper housing at an upper end, and to connect to the opening in the lower housing at a lower end. The cylindrical member may be sealingly connected to the upper and lower housings by a plurality of sealing rings and a plurality of mechanical fasteners, for example screws. When the cylindrical member is connected to the openings in the upper and lower housings, the hollow centre of the cylindrical member forms the tunnel-shaped opening through the housings.

The housing of the assembly may consist of the upper housing and the lower housing. Alternatively, the housing of the assembly may consist of the upper housing, the lower housing and the hollow cylindrical member configured to surround the opening through the housing.

### Third Aspect

According to a third aspect there is provided a surgical lighting assembly, comprising:
an upper housing and a lower housing, the upper housing and the lower housing being configured to fit together at a joint around their perimeters to define a chamber therebetween;
the lighting assembly further comprising:
   a light source in the chamber;
   a plurality of fasteners configured to fasten the upper housing and the lower housing together around their perimeters; and
   a bumper which is configured to extend around the perimeters of the upper and lower housings and to cover the plurality of fasteners and the joint between the upper and lower housings.

The plurality of fasteners are preferably a plurality of clamping strips, as described above in relation to the first aspect. The upper and lower housings preferably comprise upper and lower rims respectively, which extend around at least a portion of the housing perimeters and which are clampable by the clamping strips to hold the perimeters of the housings together in a sealed configuration.

Particularly preferably the bumper is formed from a resilient material, for example a resilient polymer material. The bumper advantageously provides shock absorption which protects the assembly from damage during use. By covering the joint between the housings and the fasteners, the bumper advantageously makes the assembly easier to clean, by preventing liquid and debris from becoming caught in and around the fasteners and the sealed joint between housings. The bumper may provide an additional layer of ingress protection, by preventing dust and liquid from coming into contact with the joint and the fasteners.

The bumper may be held in position around the perimeters of the housings by one or more rims on at least one of the housings.

In a preferred embodiment, the assembly comprises a second upper rim arranged around at least a portion of the perimeter of the upper housing, and a second lower rim arranged around at least a portion of the perimeter of the lower housing. The bumper may comprise a channel, or a pair of channels, configured to receive the second upper rim and the second lower rim. In a preferred embodiment, the bumper comprises a T-shaped channel, in which the arm of the T is configured to receive the second rims. The second rims may function to locate the bumper in position over the joint and fasteners, and to hold it in place unless enough force is applied to pull the bumper off over the second rims.

The bumper preferably comprises a fastener channel, or fastener recesses, for receiving the fasteners when the bumper extends around the outer perimeter of the housings. The fastener channel may be connected to the channel for receiving the second upper and lower rims.

It will be appreciated that features described in relation to one aspect of the present invention may also be applied equally to all of the other aspects of the present invention.

Features described in relation to the first aspect of the present invention may be applied equally to the second and third aspects of the present invention and vice versa.

### BRIEF DESCRIPTION OF DRAWINGS

The invention will be further described, by way of example only, with reference to the accompanying drawings, in which:
Figure 1 shows a partial exploded view of a surgical lighting assembly according to a preferred embodiment of an aspect of the present invention;
Figure 2 shows a partial view of the surgical lighting assembly of claim 1, including a light source and control electronics;
Figure 3 shows a perspective view of a surgical lighting assembly according to a preferred embodiment of an aspect of the present invention;
Figure 4 shows a top view of the surgical lighting assembly of Figure 3;
Figure 5 shows a bottom view of the surgical lighting assembly of Figure 3;
Figure 6 is a side-on view of the surgical lighting assembly of Figure 3; and
Figure 7 is a perspective view of a surgical lighting assembly according to a preferred embodiment of the present invention, mounted on supporting frame.

### DETAILED DESCRIPTION OF DRAWINGS

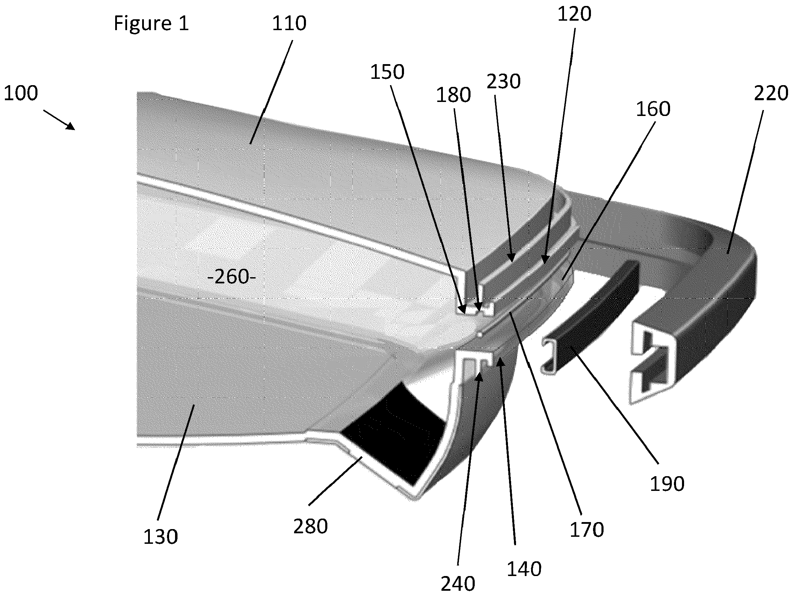

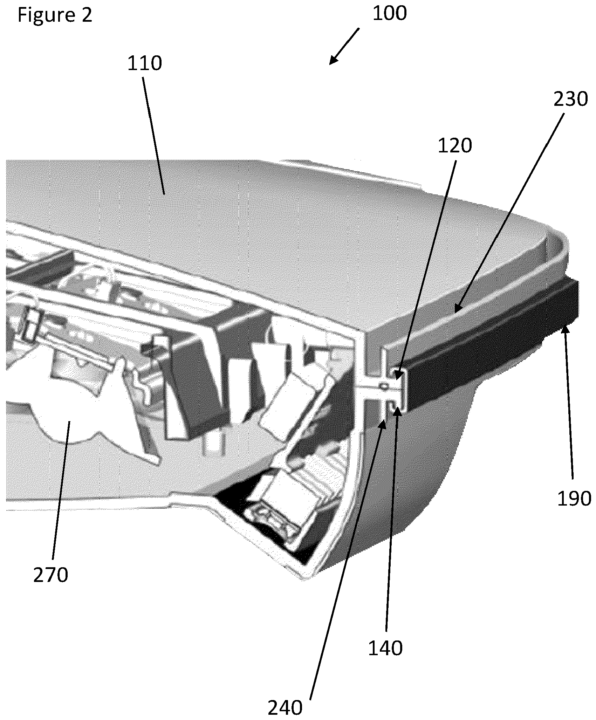

Figure 1 shows a partial exploded view of the housing of a surgical lighting assembly 100, with the internal components of the lighting assembly, for example the light source and control electronics, omitted for simplicity. Figure 2 shows the same surgical lighting assembly 100 with the light source and internal electronics included, and visible via a section taken through the lighting assembly 100.

The surgical lighting assembly 100 comprises an upper housing 110, which is formed as a one-piece moulded polymer shell. A plurality of upper rims 120 are arranged around the perimeter of the upper housing 110, with each upper rim 120 extending around a portion of a perimeter of the upper housing 110. The number and length of the upper rims may vary. In the preferred embodiment illustrated in the Figures, the upper housing 110 is hexagonal in shape, so that the perimeter of the upper housing is formed by six substantially straight sides. Each of these housing sides comprises two upper rims 120, each of the upper rims extending over a little under half of the length of each housing side, with a gap in the centre of the housing side between the ends of adjacent upper rims.

The surgical lighting assembly has a lower housing 130, which is formed as a one-piece moulded polymer shell with a perimeter shaped to match the perimeter of the upper housing 110. A plurality of lower rims 140 are arranged around the perimeter of the lower housing. The number and positions of the lower rims around the perimeter of the lower housing correspond to the positions of the upper rims relative to the perimeter of the upper housing.

The upper and lower housings comprise upper and lower flat abutment surfaces 150, 160, respectively, which extend around the housing perimeters. The upper housing and the lower housing are configured to fit together with the abutment surfaces of the two housings abutting one another around their perimeters to form a joint.

The upper and lower housings are shaped so that when the housings are fitted together in contact around their perimeters, a chamber is formed between the upper and lower housings.

A sealing member 170, in the form of a strip of resilient polymer material, is positioned between the upper and lower abutment surfaces 150, 160, and extends around the whole perimeter of the housings, so that when the abutment surfaces are in contact with one another, the sealing member is compressed between the abutment members and forms a seal.

The sealing member 170 is partially located within a sealing member channel 180 in one of the abutment surfaces. The sealing member channel 180 serves to retain the sealing member 170 in position around the perimeter of the housing, but the depth of the sealing member channel is less than the diameter of the sealing member, so that the sealing member protrudes out of the channel when uncompressed, and is reliably compressed when the abutment surfaces are brought together.

By providing the sealing member as a single strip, the step of sealing the housings together when assembling the device is greatly simplified, as only the one seal must be correctly positioned in order to seal the assembly around the entire perimeter of the housings. This advantageously increases the reliability of the sealing, as there are fewer sealed joints at which dust or water ingress could occur.

The upper and lower housings are held together by a plurality of clamping strips 190.

Each clamping strip 190 is a "profile" strip formed around a channel which fits over an upper rim and a lower rim, so that the upper and lower rims are clamped inside the clamping strip 190 and the abutment surfaces are clamped together as shown in Figure 2. In preferred embodiments, the clamping strips are metal profile strips, for example aluminium profiles, though other materials could be used. The clamping strip may comprise a channel shaped to receive the upper rim and the lower rim, and to hold the upper rim and the lower rim together inside the channel.

The upper and lower rims 120, 140 project in opposite directions away from the joint, so the clamping strip 190 comprises a generally T-shaped channel, the T-shaped channel being configured to receive the upper rim and the lower rim therein. The T-shaped channel is oriented with the arm of the T directed inwards, and the stem of the T forming the opening into the channel, so that when the clamping strip is in position, the upper and lower rims are received in the arm of the T, with the upper and lower rims pointing in opposite directions along the arm of the T-shaped channel.

The dimensions of the channel in the clamping strip 190 are selected to correspond to the dimensions of the upper and lower rims, so that the rims are held together tightly within the clamping strip.

The number of clamping strips used to hold together the housings of the surgical lighting assembly may be varied, but preferably a plurality of clamping strips 190 are employed to provide a high clamping force around substantially the whole perimeter of the housings. The number of clamping strips may correspond to the number of pairs of matching upper and lower ribs 120, 140.

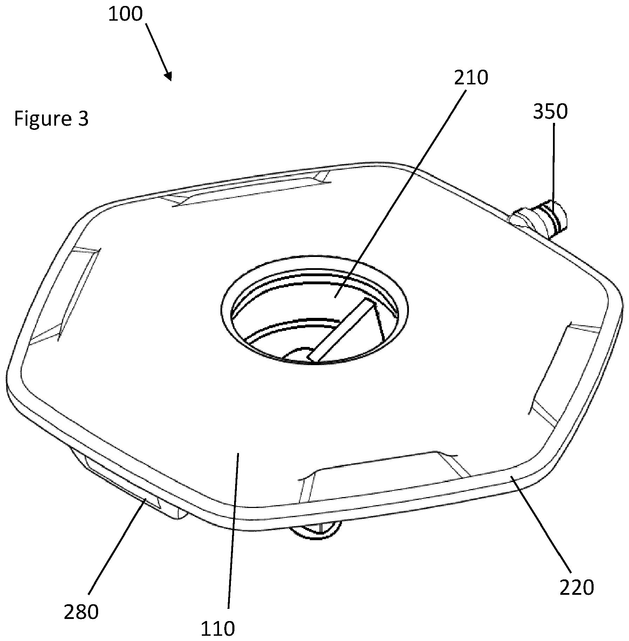

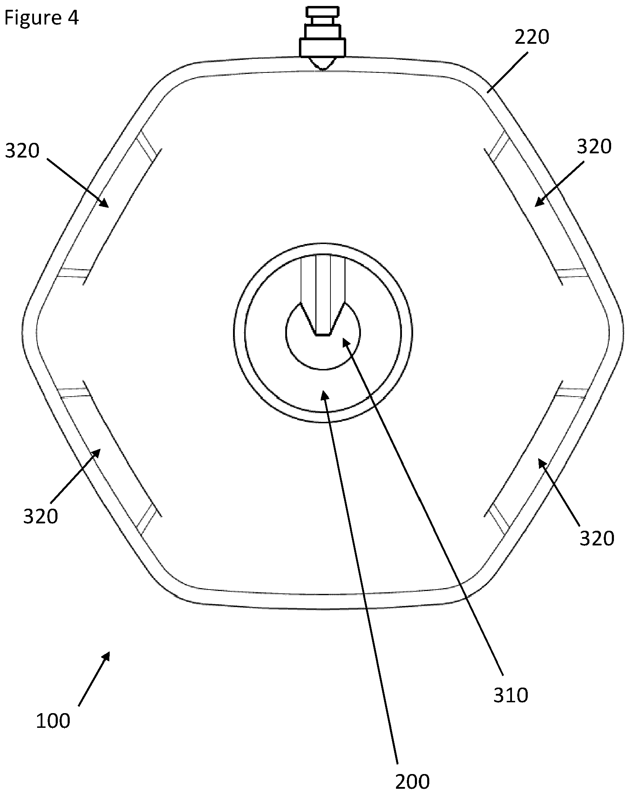

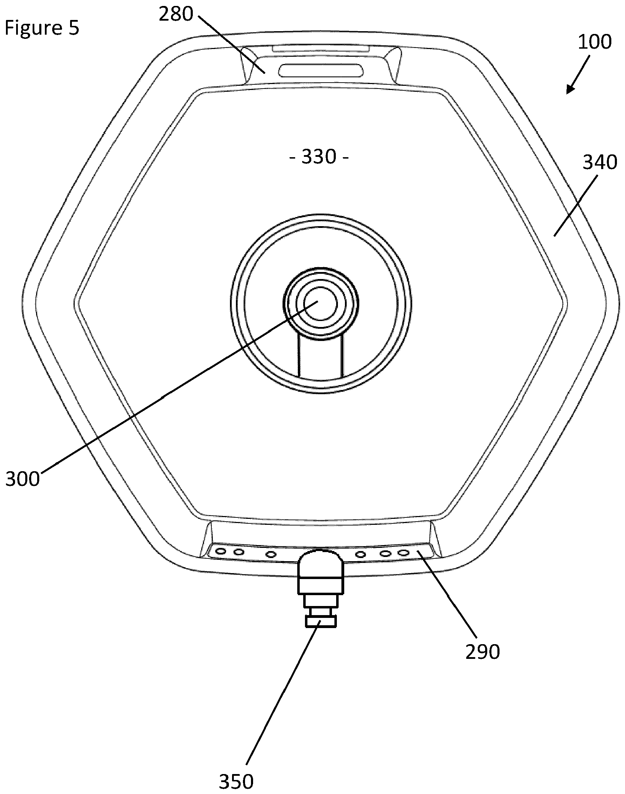

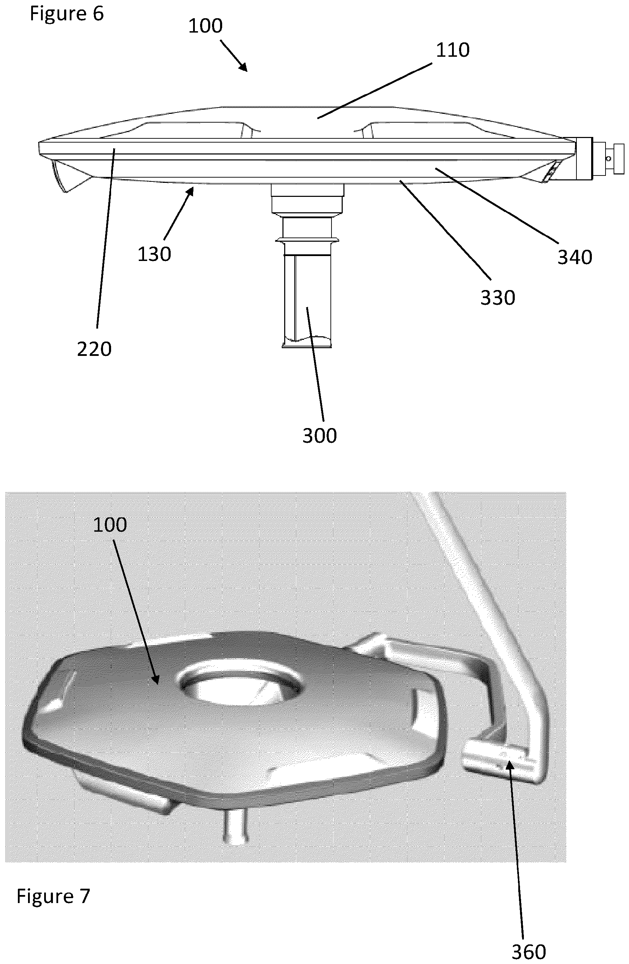

Figures 3 to 7 illustrate an embodiment of the surgical lighting assembly 100 in which the upper and lower housings are hexagonal in shape, so that the perimeter of each housing is formed by six substantially straight sides. Preferably at least one clamping strip 190 should be used to clamp the rims along each side of the hexagonal housing. In a particularly preferred embodiment, however, two clamping strips are used on each edge of the hexagonal housing, so that twelve clamping strips are spaced around the housing perimeter. The use of two clamping strips on each edge advantageously provides an extremely high and even clamping force all around the perimeter of the housing. This in turn ensures that the housing is sealed reliably all around the joint, as the clamping strips apply an even pressure to the sealing member 170 around the entire perimeter.

In the particularly preferred embodiment illustrated in the Figures, each clamping strip has a length of 122 mm, so that each clamping strips provide clamping pressure onto the housing perimeters over a distance of 122 mm. This advantageously creates a very secure clamping effect, and a high level of ingress protection.

In preferred embodiments, the upper and lower rims 120, 140, and thus the clamping positions of the plurality of clamping strips 190 are arranged symmetrically around the perimeter of the housings. A symmetrical arrangement of clamping strips advantageously produces an even clamping force around the whole perimeter of the housings, to ensure that the assembly is reliably sealed.

In a preferred embodiment, each side of the hexagonal housings comprises two upper rims and two corresponding lower rims. There is preferably a gap between adjacent pairs of rims, the ends of the rims being exposed at the gap so that a clamping strips can be slid onto the rims and moved along the rims until the pair of rims is fully received in the clamping strip.

The surgical lighting assembly illustrated in the Figures advantageously provides an IP54 rating as a result of the high level of sealing produced by the sealing member and the clamping strips around the perimeter of the upper and lower housings.

An Ingress Protection 54 (IP54) rating signifies that a lighting assembly is dust protected, such that it prevents ingress of dust sufficient to cause damage, and that it is protected against water splashing from any angle.

The outer perimeters of the upper and lower housings are only fastened together by clamping strips, and not by any other mechanical fasteners.

An opening 200 is formed in the centre of the surgical lighting assembly 100. Openings are formed in the centre of both the upper and lower housings 110, 130, and a hollow cylindrical member 210 is sealingly connected to the opening in the upper housing at an upper end, and sealingly connected to the opening in the lower housing at a lower end. The cylindrical member is connected to the lower housing by a plurality of screws (not shown) and to the upper housing by a plurality of eccentric fasteners (not shown).

The opening 200 forms a tunnel which extends through the lighthead from its top surface to its bottom surface. This opening may advantageously make the light ideal for conventional or laminar airflow systems.

A bumper 220 is formed from a continuous ring of resilient polymer material. The bumper 220 is shaped and sized to extend around the perimeters of the upper and lower housings 110, 130, and to cover the joint between the upper and lower housings

The bumper 220 is held in position around the perimeters of the housings by a second upper rim 230 which is arranged around the perimeter of the upper housing, and positioned inside the plurality of upper rims 120, and a second lower rim 240 which is arranged around the perimeter of the lower housing, inside the plurality of lower rims 140. An inside surface of the bumper 220 comprises a channel 250 which is shaped to receive the second upper rim 230, the second lower rim 240, and the clamping strips 190. The second rims function to locate the bumper 220 in position over the joint and clamping members, and to hold it in place unless enough force is applied to pull the bumper off over the second rims.

The bumper advantageously provides shock absorption which protects the assembly from damage during use. By covering the joint between the housings and the mechanical clamping strips, the bumper advantageously makes the assembly easier to clean, by preventing liquid and debris from becoming caught in and around the clamping strips and the sealed joint between housings. Although the sealing and ingress protection rating is already achieved by the sealing member and the clamping strips, the bumper may provide an additional layer of ingress protection, by preventing dust and liquid from coming into contact with the joint and the clamping strips.

In use, the upper housing 110 and the lower housing 130 are joined together by aligning the perimeters or the two housings, ensuring that the sealing member 170 is located correctly in between the upper and lower abutment surfaces 150, 160, and positioning the clamping members 190 over matching pairs of upper and lower rims 120, 140. The clamping strips automatically pull the abutment surfaces of the housings together around the housing perimeters, which overcomes any differences in shape caused by moulding tolerances during the manufacture of the upper and lower housings. This is particularly useful for upper and lower housings formed as one-piece moulded shells, such as those shown in the Figures, as the manufacturing tolerances involved in moulding such large shells can typically lead to imperfect alignments between upper and lower housings. This could lead to difficulties with sealing were conventional fasteners used, but this potential problem is overcome by the combination of the sealing member extending around the housing perimeter and the clamping strips providing a large and uniform clamping force over a large area.

When the upper and lower housings are clamped together, a chamber 260 is formed between the housings. The chamber is entirely sealed from the outside environment, as the joint around the outer perimeters of the housings is sealed by the sealing member 170, and the opening 200 through the centre of the housings is also sealed around the cylindrical member 210.

A light source 270 is arranged in the chamber 250 formed between the housings. A variety of light sources may be used, for example LED light sources known in the art for the illumination of surgical settings. The light source may comprise a plurality of LEDs. The light source is preferably configured to provide an adjustable illumination level, an adjustable pattern size, and an adjustable colour temperature of the emitted light.

A metal heat sink (not shown) is arranged in the chamber 260 on the underside of the upper housing 110. The heat sink is formed of a ring of metal attached to the underside of the upper housing, and extending around the opening 200. The heat sink comprises a plurality of cooling fins configured to distribute heat over the upper housing.

As well as the light source 270 and the heat sink, a variety of sensors and control electronics for controlling the light source are also provided inside the chamber 260.

A sensor housing 280 is moulded integrally into the lower housing 130. The sensor housing contains sensors configured to sense one or more parameters relating to the lighting provided by the light source.

A user control panel 290 is also moulded on the lower housing 130. The user control panel 290 comprises multiple control buttons through which a user can control the illumination settings of the light source 270.

An outer surface of the upper or lower housing comprises a data connection port (not shown in the Figures) connected to control electronics housed in the chamber 260. This data connection port advantageously acts as an external service interface for the electronic components inside the chamber, so that data may be exchanged, and for example software may be updated, without the need to disassemble the lighthead to access the electronic components inside. This greatly reduces the chances of dust and liquid ingress into the chamber during disassembly and reassembly, and reduces downtime for the lighting assembly.

A sterile handle 300 is attached to a mount 310 which projects into the opening 200 in the centre of the housings. When mounted on the mount, the sterile handle projects downwards underneath the lower housing 130, so that a person involved in a surgical operation, for example the surgeon, can manipulate the lighting assembly using the sterile handle.

A plurality of handles or grips 320 are moulded into the upper housing. In the illustrated embodiment, four handles 320 are provided, though other numbers are possible. The handles 320 are ridges moulded integrally into the polymer shell of the housing. These moulded handles or grips may advantageously provide non-sterile handling points so that during use of the lighting assembly in a surgical setting, attendants whose hands have not been sterilised can move the position of the lighting assembly without having to touch the sterile handle. This advantageously helps to avoid contamination caused by both sterile and non-sterile hands touching the same parts of the lighting assembly to manoeuvre the lighting assembly into the desired position. By moulding these non-sterile handles as ridges integral with the upper housing, it is ensured that these handling points are well separated from the sterile handle beneath the lower housing, and that the handles are easy to clean and do not comprise any crevices to harbour bacteria.

In the illustrated embodiment, the upper housing 110 consists of a one-piece moulded polymer shell. This advantageously reduces the number of joints and sealing points in the housing, and makes the upper housing very easy to clean and sterilise.

In the illustrated preferred embodiment, the lower housing 130 consists of a one-piece moulded polycarbonate shell. The moulded polymer shell comprises a transparent window 330 for transmitting light from the light source. By providing the lower housing as a one-piece moulded shell, the need for seals between the transparent window and a lower housing surrounding the window is eliminated. The one-piece moulded lower housing is easier to clean, simpler to assemble and disassemble with the upper housing, and provides a higher IP rating as there are fewer joints and seals for dust and liquid to enter the chamber inside the housing.

The lower housing comprises a 2k moulded polymer shell, with the transparent window 330 moulded integrally in one piece with an opaque frame 340 surrounding the transparent window.

A frame connector 350 is located on one side of the housing, the frame connector being configured to connect the lighting assembly 100 to a supporting frame 360. The supporting frame may also be called a yoke or an arm, and is preferably a multi-jointed frame assembly which is adjustable to support the lighting assembly in a variety of positions, and at a variety of angles. Suitable supporting frames 360 are known in the art.

The frame connector comprises a rotatable connection for receiving power and hard-wired data transfer from the frame. A hard-wired data connection is preferably provided as a more reliable alternative to the WLAN communications system employed in some prior art lightheads. The rotatable connection may be provided by, for example, a 9-pin connection which is rotatable around 360 degrees.

In preferred embodiments, the frame connector comprises a seal configured to prevent dust or liquid ingress at the frame connector.

In the illustrated preferred embodiment, the frame connector 350 is connected to the lower housing 130, which allows the upper housing 110 to be removable from the lower housing 130 while the lower housing is connected to the supporting frame 360.

The upper housing 110 may be removed from the lower housing 130 by removing the bumper 220, removing the clamping strips 190, and removing the fastenings around the cylindrical member 210. As the frame connector is connected to the lower housing, the upper housing may advantageously be removed without having to disconnect the entire assembly from the supporting frame. This may advantageously speed up repairs and reduce downtime, as well as reducing the risk of damage while mounting or detaching the assembly onto the support frame.

The features of the preferred surgical lighting assembly 100 described above provide a large number of benefits over similar surgical lightheads available in the prior art. For example the lighthead of the present invention provides the benefits of:
- Improved ingress protection performance, in particular reaching IP54 classification as a result of the simplified sealing and clamping around the housing perimeters;
- Simpler and more reliable sealing, thanks to the single sealing member and the even clamping pressure from the clamping strips;
- Easier and more reliable fixation of the upper and lower housings to one another, thanks to the use of the clamping strips 190;
- Being constructed from fewer parts, thanks to the use of one-piece shells for the upper and lower housings, and preferably 2K moulding for the lower housing;
- Containing fewer screws holding together the housing, which makes assembly and disassembly of the lighthead much easier and faster;
- Improved cleanability, as there are fewer gaskets and sealings, and fewer joints, making the lighthead easier to properly sterilise;
- Being more robust and better protected from damage, thanks to the resilient bumper 220 extending around the edges of the lighthead;
- The joint and fixations between the housings are hidden and protected by the bumper, making the device easier to clean, and protecting the fixations in use;
- Improved heat spreading, due to the heatsink inside the housing chamber;
- The convenient moulded handles on the upper housing can be used as non-sterile handles during surgical operations and quickly and reliably sterilised.

## Claims

1. A surgical lighting assembly, comprising:
an upper housing, with an upper rim arranged around at least a portion of a perimeter of the upper housing, and a lower housing, with a lower rim arranged around at least a portion of a perimeter of the lower housing;
the upper housing and the lower housing being configured to fit together around their perimeters to define a chamber therebetween;
the lighting assembly further comprising:
a light source in the chamber;
a sealing member located between the perimeters of the upper and lower housings; and a plurality of clamping strips;
in which the clamping strips are configured to clamp the upper rim to the lower rim around the perimeter of the housings, so that the upper housing is clamped to the lower housing in a sealed configuration.

2. A surgical lighting assembly according to claim 1, in which each clamping strip is a profile comprising a T-shaped channel, the T-shaped channel being configured to receive the upper rim and the lower rim therein.

3. A surgical lighting assembly according to claim 1 or 2, comprising a plurality of upper rims, each upper rim being arranged to extend around a portion of the perimeter of the upper housing, and/or a plurality of lower rims, each lower rim being arranged to extend around a portion of the perimeter of the lower housing.

4. A surgical lighting assembly according to claim 3, in which the plurality of upper rims extend around at least 75%, or at least 80%, or at least 85%, or at least 90% of the perimeter of the upper housing, and/or in which the plurality of lower rims extend around at least 75%, or at least 80%, or at least 85%, or at least 90% of the perimeter of the lower housing.

5. A surgical lighting assembly according to any preceding claim, in which the upper and lower housings are in the shape of regular polygons, and in which the lighting assembly comprises at least one clamping strip for each edge of the polygonal housings, preferably in which the lighting assembly comprises two or more clamping strips for each edge of the polygonal housings.

6. A surgical lighting assembly according to any preceding claim, in which the upper and lower rims and the plurality of clamping strips are arranged symmetrically around the perimeter of the housings.

7. A surgical lighting assembly according to any preceding claim, comprising a bumper which is configured to extend around the perimeters of the upper and lower housings and to cover the joint and the plurality of clamping strips, preferably in which the bumper is formed from a resilient material.

8. A surgical lighting assembly according to claim 7, in which the upper housing comprises a second upper rim arranged around at least a portion of the perimeter of the upper housing, and a second lower rim arranged around at least a portion of the perimeter of the lower housing, and in which the bumper comprises a channel configured to receive the second upper rim and the second lower rim.

9. A surgical lighting assembly according to any preceding claim, comprising a heat sink arranged in the chamber on the underside of the upper housing.

10. A surgical lighting assembly according to any preceding claim, in which an outer surface of the upper housing or the lower housing comprises a data connection port connected to control electronics housed in the chamber.

11. A surgical lighting assembly according to any preceding claim, comprising a plurality of handles on the upper housing, preferably in which the plurality of handles are ridges moulded integrally into the housing.

12. A surgical lighting assembly according to any preceding claim, in which the lower housing comprises a one-piece moulded polymer shell, the moulded polymer shell comprising a transparent window for transmitting light from the light source, preferably in which the lower housing comprises a polymer shell.

13. A surgical lighting assembly according to any preceding claim, in which the lower housing comprises a frame connector configured to connect to a supporting frame, and in which the upper housing is removable from the lower housing while the lower housing is connected to the supporting frame.

14. A surgical lighting assembly according to any preceding claim, comprising a frame connector configured to connect to a supporting frame, in which the frame connector comprises a rotatable connection for receiving power and hard-wired data transfer from the frame.

15. A surgical lighting assembly according to any preceding claim, in which the sealing member comprises a single strip of resilient material extending around the perimeters of the upper and lower housings, preferably in which the upper or lower housing comprises a peripheral channel for receiving the sealing member.
